# EUROPEAN PATENT SPECIFICATION

(11) **EP 3 518 867 B1**
(45) Date of publication and mention of the grant of the patent: **27.10.2021**
(21) Application number: 17771774.1
(22) Date of filing: 26.09.2017
(51) Int. Cl.: A61K 8/26, A46B 11/00, A61K 8/29, A61K 8/46, A61Q 11/00, A61K 8/81, A45D 34/04, A46B 15/00

(54) **TOOTH WHITENING KIT**
ZAHNWEISSUNGS-KIT
KIT DE BLANCHIMENT DES DENTS

(30) Priority: 29.09.2016 EP 16191607
(43) Date of publication of application: 07.08.2019
(73) Proprietor: Unilever IP Holdings B.V., 3013 AL Rotterdam (NL); Unilever Global IP Limited, Wirral, CH62 AZD (GB)
(72) Inventor: DELFANTI, Cristina, 00142 Roma (IT); GUOLI, Angelica, 00142 Roma (IT); POMATI, Giuseppe, 00142 Roma (IT); SALTELLI, Roberta, 00142 Roma (IT)
(74) Representative: Tansley, Sally Elizabeth
(86) International application number: PCT/EP2017/074297
(87) International publication number: WO 2018/060164

(56) References cited:
- WO-A1-2005/037127
- WO-A1-2013/070184
- WO-A1-2015/076817
- US-B1- 6 254 297
- DATABASE GNPD [Online] MINTEL; February 2009 (2009-02), "White Gloss", XP002764304, Database accession no. 1054267
- DATABASE GNPD [Online] MINTEL; July 2015 (2015-07), "Milky Flavour Toothpaste", XP002764305, Database accession no. 3284989

## Description

The present invention relates to an oral care kit that when coated onto the teeth aids tooth whitening and prevents staining.

### Background of the Invention

White teeth are seen as cosmetically attractive, however the enamel of the teeth can stain, for instance when certain foods or drinks are consumed. Tooth bleaching compositions are available but many consumers want an instance, revisable tooth whitening system. Hence there is a need for products and devices that improve the appearance of the teeth.

Peroxide based whitening compositions applied to teeth by means of bristles, a sponge surface are described in WO05037127**.**

WO2014205631 discloses an oral care composition containing (a) a zinc citrate; and (b) a surface-active organophosphate compound. The composition is said to prevent stain from depositing on a tooth surface or other oral surfaces.

Methods for reducing or preventing the staining capacity of a compound to a tooth are disclosed in WO14152829. The method includes the step of coating the tooth with a film comprising starch polymer or copolymer that is formed by crosslinking a starch.

WO 2013/070184 discloses dental whitening compositions, comprising an acrylate/octylacrylamide copolymer and one or more alkyl cellulose ethers. The compositions are said to prevent the teeth from staining.

Dental coating kits that are said to prevent teeth from staining are disclosed in EP1550428 and EP2854756.

With many tooth whitening products the teeth can appear coated with a film and the white shade of the teeth looks unnatural. There remains the need for a tooth whitening composition that gives the teeth a natural shade of white and adheres to the teeth in a manner such that the teeth do not look they are coated. The compositions of the present invention when applied to the teeth give the teeth a naturally white appearance. Application of such products can also be tricky for the consumer to apply as many formulations over time, sediment and clog applicative devices, especially application devices having a plunger based dispensing system.

The present invention relates to a whitening composition that can be applied to teeth from a dispensing system comprising a pump system, without clogging the dispensing system.

### Description of the Invention

The present invention relates to a tooth whitening kit comprising a whitening composition and an applicator;
i) the composition comprising a non-bleaching whitening agent comprising mica and a blue dye and a smectite clay comprising stearalkonium hectorite and
ii) the applicator comprising an application means, a liquid reservoir and a plunger movably mounted within the reservoir.

### Detailed Description of the Invention

The applicator of the invention comprises application means, a liquid reservoir and a plunger movably mounted within the reservoir. Preferred applicators are elongated and pen-like in shape. The reservoir of the applicator preferably contains from 0.75 ml to 5 cm³ of the tooth whitening composition.

The applicator is preferably designed so that the composition is dispensed from an aperture at one end. At the other end of the applicator is a piston/button to dispense the tooth whitening composition from the reservoir through the aperture to the applicator surface.

The plunger within the applicator is connected to the piston/button. The plunger can be manually operated by a twisting motion or by a push button. Movement of the plunger by operation of the piston/button causes the plunger to move towards the aperture and thus dispense the composition through the aperture.

It is preferable if the liquid reservoir is cylindrical.

To ensure a uniform composition it is preferable if within the liquid reservoir is a dispersal means. A preferred dispersal means is a sphere, preferably at least two spheres are present. Most preferably, the number of spheres is from 2 to 5. To ensure uniform dispersion it is preferable if the spheres comprise metal. More preferably steel.

The applicator means preferably comprises a pad, a sponge-like surface or a fibrillated surface, more preferably, it comprises a plurality of bristles.

Compositions of the invention comprise a non bleaching whitening agent comprising mica and a blue dye.

Further whitening agents include materials with a high refractive index or blue /pigments.

In the context of the present invention a high refractive index is means materials with a refractive index of at least 2.0, more preferably at least 2.2, even more preferably at least 2.3, even more preferably still at least 2.4 and most preferably at least 2.5. The maximum refractive index of the particles is not particularly limited but preferably the particles have a refractive index up to 3.0, more preferably up to 2.9. Suitable materials to provide such a high refractive index are limited only in that they should be suitable for use in oral hygiene applications. Particularly suitable are metal salts.

Preferred are salts where the metal is selected from zinc (Zn), titanium (Ti) or a combination thereof. Additionally or alternatively the metal salt is (or at least comprises) a metal oxide. Especially preferred metal oxides are zinc oxide (ZnO), titanium dioxide (TiO₂) or a combination thereof.

Preferred, owing to its high refractive index and suitability for oral application is titanium dioxide (TiO₂). Most preferred due to the natural appearance on the teeth is mica and/or mica coated with titanium dioxide.

Preferably the level of material with a high refractive index, more preferably a metal salt, in particular metal oxide such as titanium dioxide or mica is from 0.1 to 8 wt% of the total composition, more preferably it is from 0.5 to 4 wt%.

The whitening agent comprises a blue dye and mica.

A preferred blue dye is patent blue.

Preferably, the level of blue colourant, more preferably blue pigment of blue dye is from 0.0005 wt% to 0.05 wt% of the total composition, more preferably from 0.0001 wt% to 0.01 wt% The composition may comprise a mixture of whitening agents.

Preferably, the weight ratio of any of the above mentioned combinations of metal salt/mica to blue colourant, more preferably titanium dioxide/mica to blue dye, is from 2000:1 to 750:1, more preferably from 1500:1 to 1000:1.

Preferably compositions of the invention comprises less than 0.1 wt% of bleaching agent, more preferably less than 0,01 wt%, most preferably less than 0.001wt% of a bleaching agent. In the context of the present invention it is particularly advantageous to avoid bleaching agents based on peroxides.

Compositions according to the invention comprise the smectite clay stearalkonium hectorite.

Preferred compositions comprise from 5 to 25 wt% of the total composition of the above mentioned clay. More preferred compositions have 8 to 15 wt% of the total composition of the above mentioned clay.

The clay is believed to thicken the product and to adjust the appearance of the composition on the teeth so the teeth look natural. A disadvantage of the clay is that it can cause sedimentation within the product, so the level of clay should be adjusted to maximise thickening and to minimise sedimentation.

Compositions of the invention comprise an acryl based polymer, more preferably an acrylate / octylacrylamide copolymer for example a copolymer of octylacrylamide (for example N-(1, 3,3-tetramethylbutyl)-propenamide) and one or more monomers selected from acrylic acid, methacrylic acid and their simple esters, in a particular embodiment, the acrylate / ocivlacry i amide copolymer is 2-propenoic acid, 2-methyl- 2-methylpropyl ester, polymer with 2-propenoic acid and N-(1,1,3,3-tetramethylbutyl)-2-propenamide (example DERMACRYL@P 79 (commercially available from National Starch or AkzoNobel.

Preferably the level of acryl based polymer is from 2 to 20 Wt % of the total composition, more preferably from 5 to 15 wt% of the total composition.

Preferably which the weight ratio of clay to acryl based polymer is from 1.3:1 to 1:1. Compositions according to the invention will usually contain a continuous phase, which may be a mixture of water, alcohol more preferably a polyhydric alcohol. The amount of alcohol generally ranging from 20 to 85 wt% (based on the total weight of the dentifrice), preferably the level of water is 7 wt% or below and the level of alcohol is from 30 wt%. to 75 wt% of the total composition.

A preferred alcohol for use in the present invention and at the present invention is isopropyl alcohol.

In a preferred embodiment the composition comprises an ester in the continuous phase, more preferably ethyl acetate. Preferably the level of ethyl acetate is form 10 to 30 wt% of the total composition more preferably form 15 to 25 wt%.

The oral care composition of the present invention may contain a variety of other ingredients which are common in the art to enhance physical properties and performance. These ingredients include antimicrobial agents, anti-inflammatory agents, anti-caries agents, plaque buffers, fluoride sources, vitamins, plant extracts, desensitizing agents, anti- calculus agents, biomolecules, flavours, proteinaceous materials, preservatives, opacifying agents, micas, colouring agents, pH-adjusting agents, sweetening agents, particulate abrasive materials, polymeric compounds, buffers and salts to buffer the pH and ionic strength of the compositions, and mixtures thereof. Such ingredients typically and collectively makeup less than 20 percent by weight of the composition, and preferably, from 0.0 to 15 percent by weight, and most preferably, from 0.01 to 12 percent by weight of the composition, including all ranges subsumed therein.

It is highly preferable if compositions of the invention are orally acceptable. By "orally acceptable" it is meant that the compositions are safe for use in the mouth at the levels required.

Compositions of the invention preferably have a viscosity from 10,000 cps to 500,000 cps. It should be noted that in the context of the present invention all viscosities are measured at a temperature of 20 degrees centigrade with a Brookfield Viscometer, Spindle No. 4.

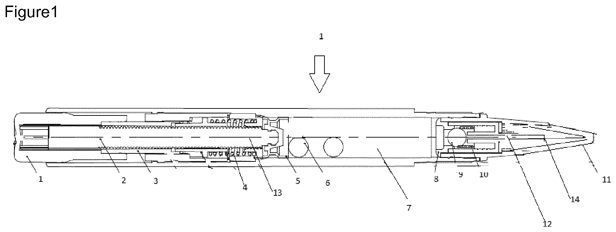

The invention will now be described by way of example only with reference to the accompanying drawing Figure 1 which is an elevational view of the dispenser in a vertical section.

Referring to the Figures, the dispenser of the invention is designated by the reference number 1. In this embodiment, dispenser comprises a barrel (7) that acts as a reservoir. At one end of the barrel is a capillary (9). Within the capillary is a ball (9) mounted between two springs (10). The capillary leads to a tip (14) which has an aperture leading to a brush (11). At the proximal end is a piston (5). The piston is attached to a screw (2) which is in contact with a sprung gearing mechanism (4). The gearing mechanism is operated by A push end cap (1). The dispenser is sealed by and plug (10) and an end cap (13). Within the barrel (5) and freely movable our two spheres (17).

Example of the invention will now be illustrated by the following non-limiting examples:

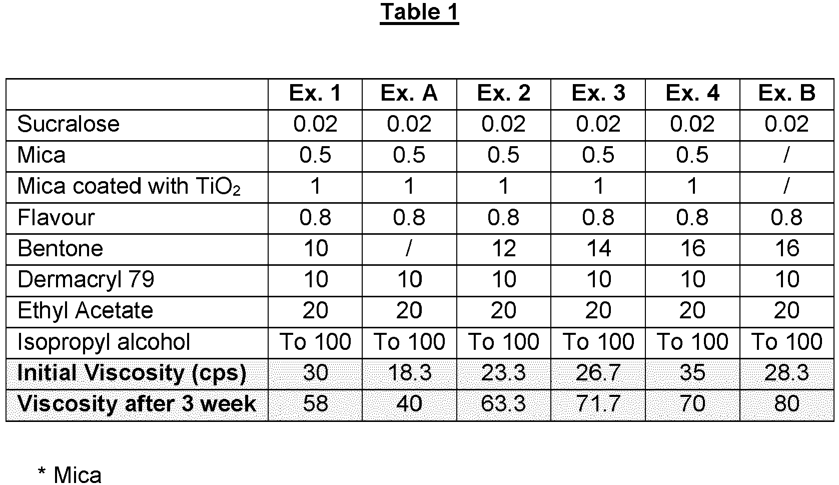

The sedimentation of the above Examples was measured and is recorded in Table 2 below

**Table 2**

| | **%SEDIMENT** |
|---|---|
| Example 1 | 1.822% |
| Example A | 2.468% |
| Example 2 | 2.3911% |
| Example 3 | 2.74495% |
| Example 4 | 3.06480% |
| Example B | 0.97921% |

Examples according to the invention coated the teeth with a natural looking film yet had acceptable sedimentation thus they did not clog the dispenser.

## Claims

1. A tooth whitening kit comprising a whitening composition and an applicator:
i) the composition comprising a non-bleaching whitening agent comprising mica and a blue dye and a smectite clay comprising stearalkonium hectorite ; and
ii) the applicator comprising an application means, a liquid reservoir and a plunger movably mounted within the reservoir.

2. A kit according to claim 1 in which the application means comprised of a plurality of bristles.

3. A kit according to any preceding claim in which comprises of at least one sphere as a dispersible means.

4. A kit according to any preceding in which the viscosity of said tooth whitening composition is from10,000 cps to 500,000 cps measured at 20°C with a Brookfield Viscometer, Spindle No. 4.

5. A kit according to any preceding claim in which the composition i) which further comprises an acryl based polymer.

6. A kit according to any claim 5 in which the acryl based polymer comprises an acrylate / octylacrylamide copolymer.

7. A kit according to claim 5 or claim 6 in which the acryl based polymer comprises 2-propenoic acid, 2-methyl-, 2-methylpropyl ester, polymer with 2-propenoic and N-(1,1,3,3-tetramethylbutyl)-2-propenamide.

8. A kit according to any one of claims 5 to 7 in which the level of acryl based polymer is from 5 to 15wt% of the total composition i).

9. A kit according to any preceding claim in which the level of clay is from 8 to 15 wt% of the total composition i).

10. A kit according to any one of claims 5 to 9 in which the weight ratio of clay to acryl based polymer is from 1.3 :1 to 1:1.

11. A kit according to any preceding claim in which the level of mica is from 0.5 to 4 wt% of the total composition.

12. A kit according to any preceding claim in which the weight ratio of mica to blue dye, is from 2000:1 to 750:1.

13. A kit according to any preceding claim which further comprises ethyl acetate.

## Patentansprüche

1. Kit zur Zahnaufhellung, umfassend eine Aufhellungszusammensetzung und einen Applikator, wobei:
i) die Zusammensetzung ein nicht bleichendes Aufhellungsmittel umfassend Glimmer und einen blauen Farbstoff und einen Smektitton umfassend Stearalkoniumhectorit umfasst; und
ii) der Applikator ein Auftragmittel, ein Flüssigkeitsreservoir und einen Kolben, der beweglich innerhalb des Reservoirs angebracht ist, umfasst.

2. Kit nach Anspruch 1, bei dem das Auftragmittel eine Vielzahl von Borsten umfasst.

3. Kit nach irgendeinem vorhergehenden Anspruch, das mindestens eine Kugel als dispergierbares Mittel umfasst.

4. Kit nach irgendeinem vorhergehenden Anspruch, bei dem die Viskosität der Zahnaufhellungszusammensetzung im Bereich von 10.000 cps bis 500.000 cps, gemessen bei 20 °C mit einem Brookfield-Viskosimeter, Spindel Nr. 4, liegt.

5. Kit nach irgendeinem vorhergehenden Anspruch, bei dem die Zusammensetzung i) ferner ein Polymer auf Acrylbasis umfasst.

6. Kit nach Anspruch 5, bei dem das Polymer auf Acrylbasis ein Acrylat/Octylacrylamid-Copolymer umfasst.

7. Kit nach Anspruch 5 oder Anspruch 6, bei dem das Polymer auf Acrylbasis 2-Propensäure, 2-Methyl-, 2-methylpropylester, Polymer mit 2-Propensäure und N-(1,1,3,3-Tetramethylbutyl)-2-propenamid umfasst.

8. Kit nach irgendeinem der Ansprüche 5 bis 7, bei dem der Gehalt an Polymer auf Acrylbasis 5 bis 15 Gew.-% der gesamten Zusammensetzung i) beträgt.

9. Kit nach irgendeinem vorhergehenden Anspruch, bei dem der Tongehalt 8 bis 15 Gew.-% der gesamten Zusammensetzung i) beträgt.

10. Kit nach irgendeinem der Ansprüche 5 bis 9, bei dem das Gewichtsverhältnis von Ton zu Polymer auf Acrylbasis 1,3:1 bis 1:1 beträgt.

11. Kit nach irgendeinem vorhergehenden Anspruch, bei dem der Glimmergehalt 0,5 bis 4 Gew.-% der gesamten Zusammensetzung beträgt.

12. Kit nach irgendeinem vorhergehenden Anspruch, bei dem das Gewichtsverhältnis von Glimmer zu blauem Farbstoff 2000:1 bis 750:1 beträgt.

13. Kit nach irgendeinem vorhergehenden Anspruch, das ferner Ethylacetat umfasst.

## Revendications

1. Kit de blanchiment des dents comprenant une composition de blanchiment et un applicateur ;
i) la composition comprenant un agent de blanchiment non-décolorant comprenant du mica et un colorant bleu et une argile de smectite comprenant de la stéaralkonium hectorite ; et
ii) l'applicateur comprenant un moyen d'application, un réservoir de liquide et un piston monté de manière détachable à l'intérieur du réservoir.

2. Kit selon la revendication 1, dans lequel le moyen d'application comprend plusieurs brosses.

3. Kit selon l'une quelconque des revendications précédentes qui comprend au moins une sphère comme un moyen dispersible.

4. Kit selon l'une quelconque des revendications précédentes, dans lequel la viscosité de ladite composition de blanchiment des dents est de 10 000 cps à 500 000 cps mesurée à 20°C avec un viscosimètre Brookfield, broche n° 4.

5. Kit selon l'une quelconque des revendications précédentes, dans lequel la composition i) comprend de plus un polymère de base acrylique.

6. Kit selon la revendication 5, dans lequel le polymère de base acrylique comprend un copolymère d'acrylate/octylacrylamide.

7. Kit selon la revendication 5 ou revendication 6, dans lequel le polymère de base acrylique comprend de l'acide 2-propénoïque, un ester 2-méthyl-, 2-méthylpropylique, un polymère avec 2-propénoïque et N-(l,l,3,3-tétraméthylbutyl)-2-propénamide.

8. Kit selon l'une quelconque des revendications 5 à 7, dans lequel la teneur en polymère de base acrylique est de 5 à 15 % en masse de la composition i) totale.

9. Kit selon l'une quelconque des revendications précédentes, dans lequel la teneur en argile est de 8 à 15 % en masse de la composition i) totale.

10. Kit selon l'une quelconque des revendications 5 à 9, dans lequel le rapport massique d'argile à polymère de base acrylique est de 1,3:1 à 1:1.

11. Kit selon l'une quelconque des revendications précédentes, dans lequel la teneur en mica est de 0,5 à 4 % en masse de la composition totale.

12. Kit selon l'une quelconque des revendications précédentes, dans lequel le rapport massique de mica à colorant bleu est de 2 000:1 à 750:1.

13. Kit selon l'une quelconque des revendications précédentes qui comprend de plus de l'acétate d'éthyle.
